# FASCICULE DE BREVET EUROPEEN

(11) **EP 3 046 899 B1**
(45) Date de publication et mention de la délivrance du brevet: **15.11.2017**
(21) Numéro de dépôt: 14761897.9
(22) Date de dépôt: 11.09.2014
(51) Int. Cl.: C07C 39/08, C07C 11/167, C07C 11/18, C09K 15/08, C07C 37/14, C07C 37/68, C07C 37/74, C08F 136/08, C08F 2/38, C08F 236/08, C07C 37/82

(54) **COMPOSITION EMPECHANT LA POLYMERISATION DE MONOMERES A INSATURATION ETHYLENIQUE ET SON ELIMINATION AVANT POLYMERISATION**
ZUSAMMENSETZUNG ZUR VERHINDERUNG DER POLYMERISIERUNG EINES ETHYLENISCH UNGESÄTTIGTEN MONOMERS UND ZUR ENTFERNUNG DAVON VOR DER POLYMERISIERUNG
COMPOSITION PREVENTING THE POLYMERIZATION OF ETHYLENICALLY UNSATURATED MONOMERS AND THE REMOVAL THEREOF BEFORE POLYMERIZATION

(30) Priorité: 19.09.2013 FR 1302178
(43) Date de publication de la demande: 27.07.2016
(73) Titulaire: Rhodia Operations, 75009 Paris (FR)
(72) Inventeur: CHRETIEN, Christelle, F-69100 Villeurbanne (FR); FISCHER, Lars, F-38200 Vienne (FR); VERDIER, Stephan, F-69008 Lyon (FR); CAVEZZAN, Jacques, F-69100 Villeurbanne (FR)
(74) Mandataire: Menville, Laure
(86) Numéro de dépôt international: PCT/EP2014/069438
(87) Numéro de publication internationale: WO 2015/039954

(56) Documents cités:
- WO-A1-98/59016
- WO-A1-03/068713

## Description

La présente invention se rapporte au domaine des compositions empêchant la polymérisation de monomères ayant une insaturation éthylénique, en particulier le butadiène et l'isoprène, ainsi qu'au domaine de l'élimination desdites compositions avant la mise en oeuvre de la polymérisation.

Le 4-tert-butylcatechol (4-TBC) est connu pour être un inhibiteur efficace empêchant la polymérisation des monomères à insaturation éthylénique. Dans l'art de la polymérisation, il est préférable de procéder à l'élimination des inhibiteurs de polymérisation de monomères avant de commencer à opérer l'étape d'amorçage de la polymérisation. En effet, la présence d'inhibiteurs dans les monomères, en cours de polymérisation, est préjudiciable car les inhibiteurs réagissent avec le catalyseur de polymérisation conduisant à des performances réduites en termes d'activité et de sélectivité. Aussi le besoin d'améliorer la capacité d'éliminer les inhibiteurs, présents dans les monomères avant d'amorcer la polymérisation, est un besoin constant. L'élimination des inhibiteurs présents dans les monomères est classiquement effectuée par distillation, par lavage au moyen d'une solution alcaline ou encore par adsorption sur un solide inorganique. Or l'une des principales contraintes pour effectuer l'élimination des inhibiteurs réside dans la composition de l'inhibiteur elle-même. Les compositions inhibitrices connues à ce jour à base de 4-TBC conduisent rarement à des performances satisfaisantes car elles nécessitent souvent, pour leur propre élimination, la mise en place de moyens conséquents, par exemple le besoin de volumes importants de solution alcaline et la nécessité de traiter les effluents lorsque l'élimination est effectuée par lavage ou encore le besoin d'une quantité importante d'adsorbant lorsque l'élimination est effectuée par adsorption. Les documents WO 03/068713 et WO 98/59015 décrivent d'autres compositions pour inhiber la polymérisation de composés éthyléniques insaturés. La présente invention se propose de remédier aux inconvénients ci-dessus par la mise à disposition d'une nouvelle composition inhibitrice à base de 4-tert-butylcatechol et présentant un profil d'impuretés déterminé. Ladite nouvelle composition inhibe efficacement la polymérisation de monomères à insaturation éthylénique. Il a été trouvé, de manière surprenante, que l'élimination de ladite composition inhibitrice selon l'invention, présente dans un monomère, est sensiblement améliorée par rapport à celle de compositions existantes sur le marché. En particulier, il a été trouvé que l'élimination de ladite composition inhibitrice selon l'invention nécessite sensiblement moins d'étapes de lavage que celles nécessitées par les compositions inhibitrices connues, ce qui est favorable en termes de volume de solution alcaline à employer et d'effluent à traiter. Il a encore été trouvé que l'élimination de ladite composition inhibitrice selon l'invention nécessite une quantité d'adsorbant sensiblement réduite par rapport à celle nécessitée par les compositions inhibitrices connues, ce qui réduit le besoin de régénérer l'adsorbant et augmente en conséquence la durée de vie de l'adsorbant.

La présente invention a pour objet une composition inhibitrice de la polymérisation de monomères à insaturation éthylénique comprenant :
- au moins 98% poids de 4-tert-butylcatechol (4-TBC),
- de 0,03% à 0,2% poids de catéchol (PC),
- au moins une impureté choisie parmi le 3-tert-butylcatechol (3-TBC), la tert-butylhydroquinone (TBHQ) et le 3,5-di-ter-butylcatechol (diTBC) et leur mélange, la quantité totale desdites impuretés et du PC représentant de 0,1% à 0,8% poids de ladite composition.

On rappelle que le catéchol correspond au 1,2-dihydroxybenzène.

Conformément à l'invention, la composition selon l'invention comprend du catéchol à titre d'impureté en une teneur comprise entre 0,03% et 0,2% poids, de préférence entre 0,03% et 0,1% poids. La composition selon l'invention comprend en outre une ou plusieurs impuretés supplémentaires choisies parmi le 3-tert-butylcatechol (3-TBC), la tert-butylhydroquinone (TBHQ) et le 3,5-di-ter-butylcatechol (diTBC). Ladite composition comprend avantageusement au moins deux desdites impuretés choisies parmi 3-TBC, TBHQ et diTBC. De manière plus préférée, elle comprend lesdites trois impuretés de sorte que la composition de l'invention comprenne du catéchol, du 3-TBC, du TBHQ et du diTBC. Avantageusement, ladite composition comprend au moins l'impureté di-TBC et au moins l'impureté PC.

Conformément à l'invention, la quantité totale des impuretés 3-TBC, PC, TBHQ et diTBC représente de 0,1% à 0,8% poids de la composition. Plus précisément, l'impureté 3-TBC, lorsqu'elle est présente dans la composition, représente de 0,02% à 0,1% poids. L'impureté TBHQ, lorsqu'elle est présente dans la composition, représente de 0,003% à 0,005% poids. L'impureté diTBC, lorsqu'elle est présente dans la composition, représente de 0,1% à 0,6% poids, de préférence de 0,15% à 0,5% poids.

Selon un mode particulier de l'invention, des additifs peuvent être ajoutés à la composition inhibitrice. La composition additivée comprend un ou plusieurs additifs choisis parmi les dispersants, les détergents, les antioxydants, les anti-mousses, les anti-rouilles et les anti-corrosions. Les additifs représentent avantageusement de 0,1% à 90% poids, de préférence de 1% à 60% poids de la composition additivée. Les dispersants sont avantageusement choisis parmi les sulfonates, les phosphates, les esters, les amines, les succinimides. Les détergents sont avantageusement choisis parmi les salicylates, les phénates, les sulfonates. Les antioxydants sont avantageusement choisis parmi les amines et dérivés du phénol. Les anti-mousses sont avantageusement choisis parmi les silicones et les acrylates. Les anti-rouilles sont avantageusement choisis parmi les amines, les esters, les dérivés du phénol, les sulfonates. Les anti-corrosions sont avantageusement choisis parmi les composés azotés comme des triazoles et des thiadiazoles. La composition additivée peut se présenter sous forme solide ou sous forme liquide. Un solvant peut être ajouté à cette composition additivée.

Conformément à l'invention, la composition à base de 4-TBC inhibe la polymérisation de monomères à insaturation éthylénique. Lesdits monomères sont avantageusement choisis parmi les mono-oléfines et les dioléfines, particulièrement les dioléfines à doubles liaisons conjuguées, les monomères vinyliques (c'est-à-dire les composés comprenant au moins un groupe vinyle), le cyclopentadiène et le dicyclopentadiène. Plus particulièrement, la composition selon l'invention est efficace pour inhiber la polymérisation du styrène et de ses dérivés du type de l'α-méthylstyrène, du vinyltoluène et du divinylbenzène, l'acide acrylique et ses esters tels que l'acrylate de méthyle, l'acrylate d'éthyle et l'acrylate de butyle, les esters de l'acide méthacrylique tels que le méthacrylate de méthyle, la méthylvinylcétone, l'acrylonitrile, l'isoprène, le 2,3-diméthylbuta-1,3-diène, le 1,3-butadiène, le chloroprène, le bromoprène, le 1-chlorobutadiène, le chlorure de vinyle et le 1,3-pentadiène. De manière plus préférée, la composition selon l'invention est avantageusement appropriée pour inhiber la polymérisation du 1,3-butadiène, de l'isoprène et du styrène.

La composition de l'invention est préparée en procédant à la réaction de catéchol avec l'isobutène ou le tert-butanol en présence d'un catalyseur acide, par exemple le bis-trifluorométhane sulfonamide, le bis-pentafluoroéthane sulfonamide et le tris-(trifluorométhane sulfonyl)-méthane. L'isobutène ou le tert-butanol est utilisé à raison de 0,01 à 50 équivalents pour 1 équivalent de catéchol. On utilise généralement une quantité de catalyseur acide inférieure ou égale à 0,01 équivalent pour 1 équivalent de catéchol. La réaction peut être conduite en phase liquide ou de manière biphasique. Elle est opérée à la pression atmosphérique, à une pression supérieure ou inférieure, et à une température comprise entre 0 et 150°C. Elle est conduite selon un procédé mis en oeuvre en discontinu ou en continu. Le mélange obtenu à l'issue de la réaction est purifié par toute méthode connue de l'Homme du métier, en particulier par extraction, par cristallisation, par distillation ou par précipitation de manière à obtenir la composition selon l'invention.

Il peut être avantageux d'ajouter un ou plusieurs solvants, par exemple le méthanol, l'eau et/ou le toluène, à la composition de l'invention. La présente invention a également pour objet une solution inhibitrice comprenant la composition inhibitrice telle que décrite ci-dessus et au moins un solvant. Le solvant peut être choisi dans le groupe consistant en le méthanol, l'eau, le toluène et leurs mélanges, par exemple un mélange de méthanol et de toluène. Le solvant peut représenter de 5% à 95% en poids de la solution inhibitrice, de façon plus préférée de 10% à 80%, et de façon encore plus préférée de 12% à 70%. Selon un mode de réalisation, la solution inhibitrice comprend :
- de 70% à 95% en poids de composition inhibitrice selon l'invention ;
- de 5% à 30% en poids d'eau ou de méthanol.
Selon un autre mode de réalisation, la solution inhibitrice comprend :
- de 35% à 65% en poids de composition inhibitrice selon l'invention ;
- de 35% à 65% en poids de toluène.
La solution inhibitrice peut en outre contenir un ou plusieurs additifs. Les additifs peuvent être choisis dans le groupe constitué par les dispersants, les détergents, les antioxydants, les anti-mousses, les anti-rouilles et les anti-corrosions.

La présente invention a encore pour objet un mélange comprenant un monomère à insaturation éthylénique et la composition selon l'invention. Ledit mélange se présente sous différentes formes, selon la forme sous lequel se trouve le monomère. Il peut s'agir d'un mélange sous forme liquide à température ambiante ou encore d'un mélange biphasique gaz/liquide.
Ledit monomère à insaturation éthylénique est choisi parmi ceux listés ci-dessus.
Ledit mélange comprend préférentiellement de 5 à 10000 ppm de ladite composition selon l'invention. Il peut encore comprendre un ou plusieurs solvants, notamment de l'eau, du toluène ou du méthanol.
Ledit mélange est stabilisé par la présence de ladite composition ayant un effet inhibiteur sur la polymérisation dudit monomère à insaturation éthylénique.

Le mélange selon l'invention peut par exemple être préparé en procédant au mélange dans n'importe quel ordre dudit monomère, de ladite composition et du solvant.

La présente invention a encore pour objet un procédé d'élimination de ladite composition inhibitrice de polymérisation selon l'invention présente dans un mélange comprenant au moins un monomère à insaturation éthylénique, ledit mélange étant celui décrit ci-dessus. Ledit procédé d'élimination est mis en oeuvre préalablement à l'emploi dudit monomère dans un procédé de polymérisation.
Un premier mode de réalisation du procédé d'élimination selon l'invention consiste à mettre en contact ladite composition inhibitrice de polymérisation de monomères à insaturation éthylénique avec au moins un oxyde inorganique solide. Ledit oxyde organique joue le rôle d'adsorbant sur lequel ladite composition inhibitrice s'adsorbe. Ledit procédé d'élimination selon ledit premier mode de réalisation repose donc sur le principe d'adsorption. Ledit oxyde organique est préférentiellement choisi parmi les alumines, les silices et les silices-alumines. De manière préférée, ledit oxyde organique est une alumine. Tout type d'alumine convient pour la mise en oeuvre dudit procédé, en particulier des alumines microporeuses, mésoporeuses ou macroporeuses. Les différents types de structures cristallines alpha, beta et gamma sont également appropriées.
De manière avantageuse, ledit procédé d'adsorption est opéré à température ambiante. Ledit oxyde inorganique est préférentiellement préalablement soumis à un traitement thermique à une température avantageusement comprise entre 200 et 400°C.

Les performances dudit premier mode de réalisation du procédé d'élimination selon l'invention se mesurent par la mise en évidence d'un point de rupture correspondant à la durée à partir de laquelle l'adsorbant commence à ne plus remplir son rôle d'adsorbant. Plus le point de rupture est élevé, plus ledit procédé d'élimination par adsorption est efficace c'est-à-dire qu'il nécessite une quantité réduite d'adsorbant par rapport à celle nécessitée par une composition inhibitrice antérieure pour éliminer une même quantité de composition inhibitrice.

Un deuxième mode de réalisation du procédé d'élimination selon l'invention consiste à procéder au lavage avec une solution alcaline dudit mélange comprenant au moins un monomère à insaturation éthylénique et ladite composition inhibitrice de polymérisation. De manière préférée, ladite solution alcaline est une solution aqueuse d'hydroxyde de sodium, notamment de la soude caustique, ou une solution aqueuse d'hydroxyde de potassium.

Ledit procédé d'élimination selon ledit deuxième mode de réalisation repose donc sur le principe d'absorption de la composition inhibitrice par lavage, ladite composition se retrouvant, après séparation des phases organique et aqueuse, dans ladite phase aqueuse formée de ladite solution alcaline.

Les performances dudit deuxième mode de réalisation du procédé d'élimination selon l'invention se mesurent par le nombre de lavage pouvant être opéré par la même solution alcaline sans que la composition inhibitrice reste dans le monomère.

Un troisième mode de réalisation du procédé d'élimination selon l'invention consiste à procéder à la distillation dudit mélange. La distillation dudit mélange est conduite de manière classique par l'Homme du métier.

La présente invention a encore pour objet un procédé de polymérisation de monomères à insaturation éthylénique comprenant une étape d'élimination de ladite composition inhibitrice mise en oeuvre selon l'un des modes de réalisation décrits ci-dessus suivie d'au moins une étape d'amorçage de polymérisation. Ledit procédé de polymérisation est mis en oeuvre de manière conventionnelle dans des conditions classiques connues de l'Homme du métier versé dans la polymérisation de monomères à insaturation éthylénique.

L'invention sera mieux comprise à la lumière des exemples suivants proposés à titre d'illustration.

### Exemple 1 : préparation de différentes compositions inhibitrices de polymérisation

Dans un réacteur de 170 ml, on introduit successivement 50 g de catéchol et une quantité catalytique de bis-trifluorométhane sulfonamide. Le mélange réactionnel est purgé à l'azote, chauffé à température de 120°C puis mis sous agitation. Une pression de 1 bar d'isobutène est alors appliquée et la réaction est arrêtée lorsque environ 11 g d'isobutène ont été consommés. A l'issue de la réaction, le mélange obtenu est purifié dans des conditions telles qu'elles permettent d'obtenir les différentes compositions A, B et C. Les compositions A, B et C contiennent du 4-TBC et les impuretés décrites dans le tableau 1 ci-dessous.

| | Teneur totale en impuretés | PC (% poids) | 3-TBC (% poids) | diTBC (% poids) | TBHQ (% poids) |
|---|---|---|---|---|---|
| Composition A (invention) | 0,70 | 0,1 | 0,1 | 0,5 | 0,0045 |
| Composition B (comparatif) | 0,62 | 0,009 | 0,01 | 0,60 | 0,002 |
| Composition C (comparatif) | 0,861 | 0,001 | 0,15 | 0,65 | 0,06 |

La performance d'inhibition des compositions A, B et C a été validée sur l'isoprène :
3 tubes de 10 ml ont été remplis avec une solution d'isoprène et 20 ppm de TBC, apporté respectivement par les compositions A, B et C. Les tubes ont été sellés et introduit dans un bain à 100°C pendant 16 heures. Après 16 heures, l'isoprène a été évaporé de chaque tube à 50°C et séché à 35°C sous pression réduite. Le taux de conversion de l'isoprène en polymère a été déterminé dans chaque tube.
On a constaté que ces taux de conversion étaient équivalents dans les trois tubes. Les compositions A, B et C ont donc des performances d'inhibition comparables.

### Exemple 2 : Elimination des compositions inhibitrices A, B et C par lavage à la soude

Chacune desdites compositions A, B et C est présente dans un mélange à base d'isoprène.

Le protocole opératoire pour conduire l'élimination de chacune desdites compositions A, B et C présente dans l'isoprène est le suivant :
1- On introduit 3 g d'une solution d'hydroxyde de sodium dans une ampoule à décanter.
2- On ajoute 10 g d'une solution isoprène-composition inhibitrice, la teneur de la composition inhibitrice étant égale à 1000 ppm.
3- Après agitation, l'ensemble est mis à décanter jusqu'à ce que deux phases se séparent : la phase organique contenant l'isoprène en haut et la phase aqueuse avec la composition inhibitrice en bas.
4- On effectue un prélèvement de la phase supérieure, lequel est analysé par chromatographie en phase liquide haute performance (HPLC). On mesure la quantité de 4-TBC dans l'isoprène.
5- La phase aqueuse à base d'hydroxyde de sodium est récupérée pour la recycler dans une nouvelle étape de lavage.
6- Cette procédure est reproduite en utilisant à chaque étape de lavage la même solution d'hydroxyde de sodium que celle utilisée lors de la première étape de lavage (recyclage de la solution d'hydroxyde de sodium) et une nouvelle solution d'isoprène-composition inhibitrice ayant la même formulation que celle précisée au point 2 ci-dessus. Cette procédure est reproduite jusqu'à ce que la solution d'hydroxyde de sodium ne soit plus efficace pour éliminer la composition inhibitrice de l'isoprène.

Les résultats obtenus avec les compositions inhibitrices A, B et C figurent dans le tableau 2 ci-dessous.

**Tableau 2 : performance de l'élimination des compositions inhibitrices A, B et C par lavage à l'hydroxyde de sodium**

| Composition inhibitrice | A (invention) | B (comparatif) | C (comparatif) |
|---|---|---|---|
| Nombre d'étapes de lavage avec la même solution de NaOH | 1,2 N | N | N |

Les compositions inhibitrices B et C permettent de réaliser N étapes de lavage efficacement. Les résultats démontrent que le nombre d'étapes de lavage avec la même solution de NaOH est augmenté de 20% en utilisant la composition inhibitrice A. Ainsi, l'utilisation de la composition inhibitrice A permet de limiter les volumes de solution alcaline mis en oeuvre pour éliminer la composition inhibitrice, particulièrement le 4-TBC, présent(e) dans l'isoprène.

### Exemple 4 : Elimination des compositions inhibitrices A, B et C par adsorption sur alumine

Chacune desdites compositions A, B et C est présente dans un mélange à base d'isoprène.

Le protocole opératoire pour conduire l'élimination de chacune desdites compositions A, B et C présente dans l'isoprène est le suivant :
1- On introduit 50 g d'alumine (AxSorb 920, Axens) dans une colonne en verre.
2- Une fois que l'alumine est bien tassée, on introduit une solution d'isoprène (300 ml) au-dessus de l'alumine. Le niveau supérieur de la solution d'isoprène est repéré de manière à ce qu'il reste inchangé durant toute la durée du test.
3- On introduit une solution d'isoprène contenant 2% poids d'une composition inhibitrice (A, B ou C) avec un débit égal à 1,1 ml/min.
4- On règle le flux d'isoprène en sortie de colonne de sorte à maintenir toujours au même niveau la solution d'isoprène en entrée de colonne.

On prélève régulièrement un échantillon de solution d'isoprène en sortie de colonne de manière à l'analyser par chromatographie en phase liquide haute performance (HPLC). On mesure ainsi la quantité de composition inhibitrice, en particulier de 4-TBC, dans la solution d'isoprène recueillie au cours du temps. Le temps à partir duquel la quantité de 4-TBC augmente sensiblement dans la solution d'isoprène analysée correspond à un point de rupture au-delà duquel l'alumine commence à ne plus remplir son rôle d'adsorbant.

Les résultats obtenus avec les compositions inhibitrices A, B et C figurent dans le tableau 3 ci-dessous.

**Tableau 3 : performance de l'élimination des compositions inhibitrices A, B et C par adsorption sur alumine**

| Composition inhibitrice | A (invention) | B (comparatif) | C (comparatif) |
|---|---|---|---|
| Point de rupture (durée) | 1,66 T | T | T |

Au-delà du temps T, les compositions inhibitrices B et C ne sont plus efficacement adsorbées sur l'alumine.

Les résultats démontrent que la capacité d'adsorption de l'alumine est sensiblement supérieure en utilisant la composition inhibitrice A. Ainsi, la régénération de l'alumine peut être davantage espacée et la durée de vie de l'adsorbant est rallongée par l'emploi de la composition inhibitrice A.

## Revendications

1. Composition inhibitrice de la polymérisation de monomères à insaturation éthylénique comprenant :
- au moins 98% poids de 4-tert-butylcatechol (4-TBC),
- de 0,03% à 0,2% poids de catéchol (PC),
- au moins une impureté choisie parmi le 3-tert-butylcatechol (3-TBC), la tert-butylhydroquinone (TBHQ) et le 3,5-di-ter-butylcatechol (diTBC) et leur mélange, la quantité totale desdites impuretés et du PC représentant de 0,1% à 0,8% poids de ladite composition.

2. Composition inhibitrice selon la revendication 1 dans laquelle l'impureté 3-TBC représente de 0,02% à 0,1% poids.

3. Composition inhibitrice selon la revendication 1 ou la revendication 2 dans laquelle le catéchol (PC) représente de 0,03% à 0,1% poids.

4. Composition inhibitrice selon l'une des revendications 1 à 3 dans laquelle l'impureté TBHQ représente de 0,003% à 0,005% poids.

5. Composition inhibitrice selon l'une des revendications 1 à 4 dans laquelle l'impureté diTBC représente de 0,1% à 0,6% poids.

6. Composition inhibitrice selon l'une des revendications 1 à 5 dans laquelle l'impureté diTBC représente de 0,15% à 0,5% poids.

7. Composition inhibitrice selon l'une des revendications 1 à 6 telle qu'elle comprend un ou plusieurs additifs choisis parmi les dispersants, les détergents, les antioxydants, les anti-mousses, les anti-rouilles et les anti-corrosions.

8. Solution inhibitrice de la polymérisation de monomères à insaturation éthylénique comprenant la composition selon l'une quelconque des revendications 1 à 7 et au moins un solvant.

9. Solution selon la revendication 8, **caractérisée en ce que** le solvant est choisi dans le groupe consistant en le méthanol, l'eau, le toluène et leurs mélanges.

10. Mélange comprenant un monomère à insaturation éthylénique et la composition selon l'une des revendications 1 à 7.

11. Mélange selon la revendication 10 dans lequel ledit monomère est choisi parmi les mono-oléfines et les dioléfines, les monomères vinyliques, le cyclopentadiène et le dicyclopentadiène.

12. Procédé d'élimination de ladite composition inhibitrice de polymérisation présente dans un mélange comprenant au moins un monomère à insaturation éthylénique selon la revendication 10 ou la revendication 11.

13. Procédé d'élimination selon la revendication 12 tel qu'il consiste à mettre en contact ladite composition inhibitrice de polymérisation de monomères à insaturation éthylénique avec au moins un oxyde inorganique solide.

14. Procédé d'élimination selon la revendication 12 tel qu'il consiste à procéder au lavage avec une solution alcaline dudit mélange comprenant au moins un monomère à insaturation éthylénique et ladite composition inhibitrice de polymérisation.

15. Procédé d'élimination selon la revendication 12 tel qu'il consiste à procéder à la distillation dudit mélange.

16. Procédé de polymérisation de monomères à insaturation éthylénique comprenant une étape d'élimination de ladite composition inhibitrice selon le procédé de l'une des revendications 12 à 15 suivie d'au moins une étape d'amorçage de polymérisation.

## Patentansprüche

1. Zusammensetzung, die die Polymerisation von ethylenisch ungesättigten Monomeren hemmt und Folgendes umfasst:
- mindestens 98 Gew.-% 4-tert-Butylbrenzcatechin (4-TBC),
- 0,03 Gew.-% bis 0,2 Gew.-% Brenzcatechin (BC),
- mindestens eine Verunreinigung, ausgewählt aus 3-tert-Butylbrenzcatechin (3-TBC), tert-Butylhydrochinon (TBHC) und 3,5-Di-tert-Butylbrenzcatechin (diTBC) und ihrer Mischung, wobei die Gesamtmenge der Verunreinigungen und von BC 0,1 Gew.-% bis 0,8 Gew.-% der Zusammensetzung ausmacht.

2. Hemmende Zusammensetzung nach Anspruch 1, wobei die 3-TBC-Verunreinigung 0,02 Gew.-% bis 0,1 Gew.-% ausmacht.

3. Hemmende Zusammensetzung nach Anspruch 1 oder Anspruch 2, wobei Brenzcatechin (BC) 0,03 Gew.-% bis 0,1 Gew.-% ausmacht.

4. Hemmende Zusammensetzung nach einem der Ansprüche 1 bis 3, wobei die TBHC-Verunreinigung 0,003 Gew.-% bis 0,005 Gew.-% ausmacht.

5. Hemmende Zusammensetzung nach einem der Ansprüche 1 bis 4, wobei die diTBC-Verunreinigung 0,1 Gew.-% bis 0,6 Gew.-% ausmacht.

6. Hemmende Zusammensetzung nach einem der Ansprüche 1 bis 5, wobei die diTBC-Verunreinigung 0,15 Gew.-% bis 0,5 Gew.-% ausmacht.

7. Hemmende Zusammensetzung nach einem der Ansprüche 1 bis 6, die einen oder mehrere Zusatzstoffe umfasst, ausgewählt aus Dispersionsmitteln, Reinigungsmitteln, Antioxidationsmitteln, Entschäumern, Rostschutz- und Korrosionsschutzmitteln.

8. Lösung, die die Polymerisation von ethylenisch ungesättigten Monomeren hemmt und die Zusammensetzung nach einem der Ansprüche 1 bis 7 und mindestens ein Lösungsmittel umfasst.

9. Lösung nach Anspruch 8, **dadurch gekennzeichnet, dass** das Lösungsmittel aus der Gruppe bestehend aus Methanol, Wasser, Toluol und ihren Mischungen ausgewählt ist.

10. Mischung, die ein ethylenisch ungesättigtes Monomer und die Zusammensetzung nach einem der Ansprüche 1 bis 7 umfasst.

11. Mischung nach Anspruch 10, wobei das Monomer aus Monoolefinen und Diolefinen, Vinylmonomeren, Cyclopentadien und Dicyclopentadien ausgewählt ist.

12. Verfahren zur Entfernung der polymerisationshemmenden Zusammensetzung, die in einer Mischung, die mindestens ein ethylenisch ungesättigtes Monomer umfasst, nach Anspruch 10 oder Anspruch 11 vorliegt.

13. Entfernungsverfahren nach Anspruch 12, das darin besteht, die Zusammensetzung, die die Polymerisation von ethylenisch ungesättigten Monomeren hemmt, mit mindestens einem anorganischen Festoxid in Kontakt zu bringen.

14. Entfernungsverfahren nach Anspruch 12, das darin besteht, die Mischung, die mindestens ein ethylenisch ungesättigtes Monomer und die polymerisationshemmende Zusammensetzung umfasst, mit einer alkalischen Lösung zu waschen.

15. Entfernungsverfahren nach Anspruch 12, das darin besteht, die Mischung zu destillieren.

16. Verfahren zur Polymerisierung von ethylenisch ungesättigten Monomeren, das einen Schritt zur Entfernung der hemmenden Zusammensetzung gemäß dem Verfahren nach einem der Ansprüche 12 bis 15 gefolgt von mindestens einem Schritt zur Polymerisationsinitiierung umfasst.

## Claims

1. Composition for inhibiting the polymerization of ethylenically unsaturated monomers, comprising:
- at least 98% by weight of 4-tert-butylcatechol (4-TBC),
- from 0.03% to 0.2% by weight of catechol (PC),
- at least one impurity chosen from 3-tert-butylcatechol (3-TBC), tert-butylhydroquinone (TBHQ) and 3,5-di-tert-butylcatechol (diTBC) and a mixture thereof, the total amount of said impurities and of the PC representing from 0.1% to 0.8% by weight of said composition.

2. Inhibitor composition according to Claim 1, in which the impurity 3-TBC represents from 0.02% to 0.1% by weight.

3. Inhibitor composition according to Claim 1 or Claim 2, in which catechol (PC) represents from 0.03% to 0.1% by weight.

4. Inhibitor composition according to one of Claims 1 to 3, in which the impurity TBHQ represents from 0.003% to 0.005% by weight.

5. Inhibitor composition according to one of Claims 1 to 4, in which the impurity diTBC represents from 0.1% to 0.6% by weight.

6. Inhibitor composition according to one of Claims 1 to 5, in which the impurity diTBC represents from 0.15% to 0.5% by weight.

7. Inhibitor composition according to one of Claims 1 to 6, such that it comprises one or more additives chosen from dispersants, detergents, antioxidants, antifoams, rust inhibitors and corrosion inhibitors.

8. Solution for inhibiting the polymerization of ethylenically unsaturated monomers, comprising the composition according to any one of Claims 1 to 7 and at least one solvent.

9. Solution according to Claim 8, **characterized in that** the solvent is chosen from the group consisting of methanol, water and toluene, and mixtures thereof.

10. Mixture comprising an ethylenically unsaturated monomer and the composition according to one of Claims 1 to 7.

11. The mixture according to Claim 10, in which said monomer is chosen from monoolefins and diolefins, vinyl monomers, cyclopentadiene and dicyclopentadiene.

12. Process for removing said polymerization-inhibiting composition present in a mixture comprising at least one ethylenically unsaturated monomer according to Claim 10 or Claim 11.

13. Removal process according to Claim 12, such that it consists in placing said composition for inhibiting the polymerization of ethylenically unsaturated monomers in contact with at least one solid inorganic oxide.

14. Removal process according to Claim 12, such that it consists in washing with an alkaline solution said mixture comprising at least one ethylenically unsaturated monomer and said polymerization-inhibiting composition.

15. Removal process according to Claim 12, such that it consists in distilling said mixture.

16. Process for polymerizing ethylenically unsaturated monomers, comprising a step of removal of said inhibitor composition according to the process of one of Claims 12 to 15, followed by at least one step of initiating polymerization.
